(19) Europäisches Patentamt
European Patent Office
Office européen des brevets

(11) Veröffentlichungsnummer: **0 237 902**
**A2**

(12)

# EUROPÄISCHE PATENTANMELDUNG

(21) Anmeldenummer: **87103347.8**

(22) Anmeldetag: **09.03.87**

(51) Int. Cl.³: **C 07 D 249/08**

(30) Priorität: **19.03.86 DE 3609152**

(43) Veröffentlichungstag der Anmeldung:
**23.09.87 Patentblatt 87/39**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kraatz, Udo, Dr.**
**Andreasstrasse 22a**
**D-5090 Leverkusen 1(DE)**

(54) **Verfahren zur Herstellung des (-)-Antipoden des (E)-1-Cyclohexyl-4,4-dimethyl-3-hydroxy-2-(1,2,4-triazol-1-yl)-pent-1-ens.**

(57) Nach einem neuen Verfahren läßt sich der (−)-Antipode des (E)-1-Cyclohexyl-4,4-dimethyl-3-hydroxy-2-(1,2,4-triazol-1-yl)-pent-1-éns herstellen, indem man das (E)-Isomere des 1-Cyclohexyl-4,4-dimethyl-2-(1,2,4-triazol-1-yl)-pent-1-en-3-ons

a) mit Borhydrid in Gegenwart eines optisch aktiven Prolin-Derivates der Formel

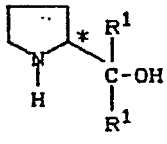

(III)

in welcher

$R^1$ für Alkyl, Phenyl oder Benzyl steht, oder

b) mit komplexen Borhydriden in Gegenwart eines Säure-additions-Salzes eines optisch aktiven Prolin-Derivates der Formel (III)

umsetzt.

Die optisch aktiven Prolin-Derivate der Formel (III) sowie deren Säureadditions-Salze sind neu.

EP 0 237 902 A2

0237902

BAYER AKTIENGESELLSCHAFT 5090 Leverkusen, Bayerwerk
Konzernverwaltung RP
Patentabteilung Dü/ABc
IVa

Verfahren zur Herstellung des (-)-Antipoden des (E)-1-Cyclohexyl-4,4-dimethyl-3-hydroxy-2-(1,2,4-triazol-1-yl)-pent-1-ens

Die vorliegende Erfindung betrifft ein neues Verfahren zur Herstellung des bekannten (-)-Antipoden*) des (E)-1-Cyclohexyl-4,4-dimethyl-3-hydroxy-2-(1,2,4-triazol-1-yl)-pent-1-ens.

Es ist bereits bekannt geworden, den (-)-Antipoden des (E)-1-Cyclohexyl-4,4-dimethyl-3-hydroxy-2-(1,2,4-triazol-1-yl)-pent-1-ens dadurch herzustellen, daß man die entsprechende racemische Verbindung mit einem optisch aktiven Säurechlorid umsetzt, das entstehende Ester-Diastereomeren-Gemisch auf chromatographischem Wege trennt und den Ester, der den (-)-Antipoden enthält, verseift (vgl. DE-OS 33 02 122). Nachteilig an diesem Verfahren ist aber

*) Unter dem (-)-Antipoden ist hier jeweils dasjenige Enantiomere zu verstehen, das die Schwingungsebene von linear polarisiertem Licht der Natrium-D-Linie nach links dreht.

daß es nur zur Synthese geringer Mengen des gewünschten Antipoden geeignet ist.

Weiterhin ist bereits bekannt geworden, daß man Ketone mit Reduktionsmitteln in Gegenwart verschiedener chiraler Hilfsreagentien zu optisch aktiven Carbinolen reduzieren kann (vgl. Chem. Pharm. Bull. 31 837 (1983) und EP-OS 0 054 431). Unbefriedigend ist jedoch, daß dieses Verfahren nicht generell anwendbar ist. So lassen sich Ketone, die keine aromatischen Gruppen enthalten, nur in Carbinole mit einer für praktische Zwecke nicht ausreichenden optischen Reinheit überführen.

Außerdem ist bekannt, daß sich der (-)-Antipode des (E)-1-Cyclohexyl-4,4-dimethyl-3-hydroxy-2-(1,2,4-triazol-1-yl)-pent-1-ens synthetisieren läßt, indem man das (E)-Isomere des 1-Cyclohexyl-4,4-dimethyl-2-(1,2,4-triazol-1-yl)-pent-1-en-3-ons mit Borhydriden in Gegenwart von (-)-Norephedrin reduziert (vgl. EP-OS 142 566). Ein entscheidender Nachteil dieses Verfahrens besteht jedoch darin, daß die optische Reinheit des gewünschten Produktes relativ gering ist.

Es wurde nun gefunden, daß man den (-)-Antipoden des (E)-1-Cyclohexyl-4,4-dimethyl-3-hydroxy-2-(1,2,4-triazol-1-yl)-pent-1-ens der Formel

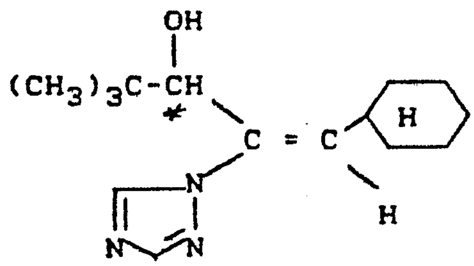

(I)

erhält, indem man (E)-Isomeres des 1-Cyclohexyl-4,4-dimethyl-2-(1,2,4-triazol-1-yl)-pent-1-en-3-ons der Formel

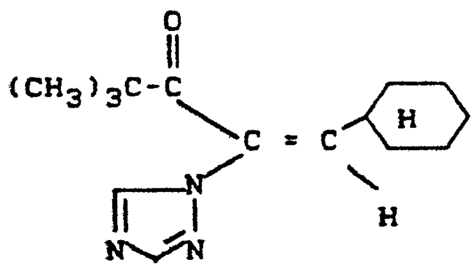

(II)

a) mit Borhydrid in Gegenwart eines optisch aktiven Prolin-Derivates der Formel

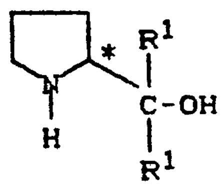

(III)

in welcher

$R^1$ für Alkyl, Phenyl oder Benzyl steht,

oder

b) mit komplexen Borhydriden in Gegenwart eines Säureadditions-Salzes eines optisch aktiven Prolin-Derivates der Formel (III) in Gegenwart eines Verdünnungsmittels bei Temperaturen zwischen -70°C und +60°C umsetzt.

Es ist als äußerst überraschend zu bezeichnen, daß sich der (-)-Antipode des (E)-1-Cyclohexyl-4,4-dimethyl-3-hydroxy-2-(1,2,4-triazol-1-yl)-pent-1-ens der Formel (I) nach dem erfindungsgemäßen Verfahren in sehr hoher Ausbeute und ausgezeichneter optischer Reinheit darstellen läßt, denn aufgrund des bekannten Standes der Technik konnte nicht damit gerechnet werden, daß die Umsetzung selektiv zu dem gewünschten Produkt führt. Vor allen Dingen war nicht zu erwarten, daß die Umsetzung auch bei relativ hohen Temperaturen nahezu frei von Nebenreaktionen abläuft.

Das erfindungsgemäße Verfahren zeichnet sich durch eine Reihe von Vorteilen aus. So sind die Reaktionskomponenten auch in größeren Mengen zugänglich und auch im technischen Maßstab problemlos zu handhaben. Ferner ist der zur Durchführung des Verfahrens erforderliche apparative Aufwand gering, und die Aufarbeitung des nach beendeter Umsetzung anfallenden Reaktionsgemisches bereitet keine Schwierigkeiten. Insbesondere jedoch läßt sich der (-)-Antipode des (E)-1-Cyclohexyl-4,4-dimethyl-3-hydroxy-2-(1,2,4-triazol-1-yl)-pent-1-ens der Formel (I) nach dem erfindungsgemäßen Verfahren in höherer Ausbeute und besserer optischer Reinheit herstellen als nach den bisher bekannten Methoden.

Der nach dem erfindungsgemäßen Verfahren herstellbare (-)-Antipode des (E)-1-Cyclohexyl-4,4-dimethyl-3-hydroxy-2-(1,2,4-triazol-1-yl)-pent-1-ens ist durch die Formel (I) eindeutig chrakterisiert. In dieser Formel ist das asymmetrisch substituierte Kohlenstoffatom, welches das Chiralitätszentrum darstellt, durch ein (*) markiert. Durch den Buchstaben "E" vor dem systematischen Namen der Verbindung der Formel (I) wird ausgedrückt, daß der Cyclohexyl-Rest und der 1,2,4-Triazolyl-Rest auf <u>entgegengesetzten</u> Seiten der Doppelbindung stehen.

Verwendet man Borhydrid als Reduktionsmittel, S-2-(Dibenzyl-hydroxymethyl)-pyrrolidin als chirales Hilfsreagenz und Tetrahydrofuran (= THF) als Verdünnungsmittel, so kann der Verlauf des erfindungsgemäßen Verfahrens (Variante a) durch das folgende Formelschema wiedergegeben werden:

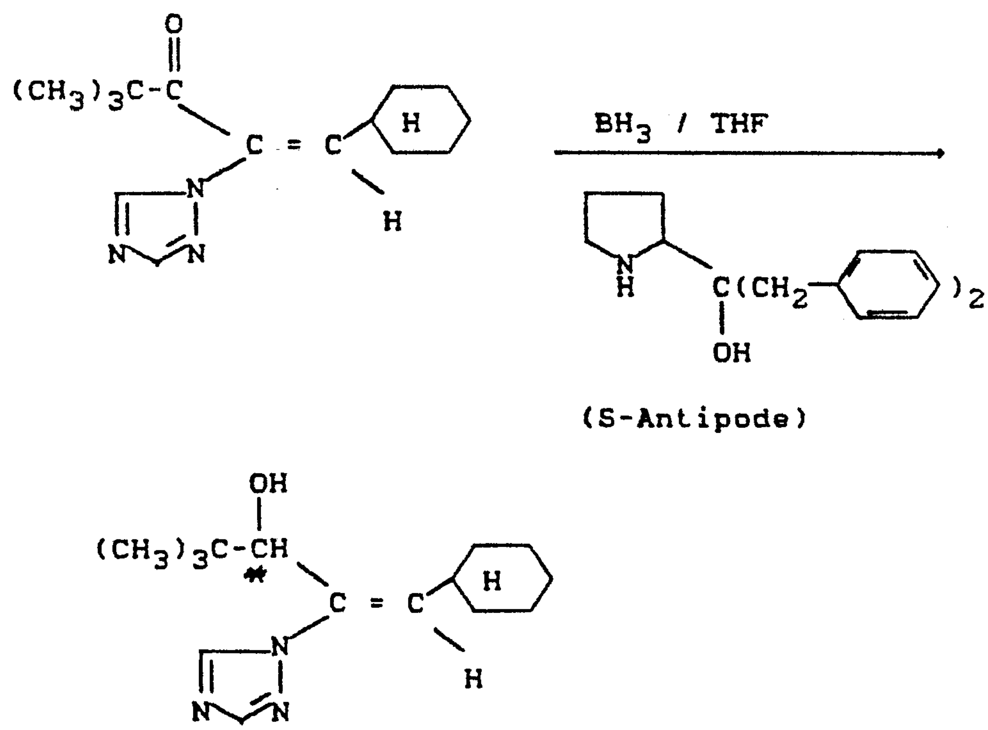

Verwendet man Natriumboranat als Reduktionsmittel und das Hydrochlorid des S-2-(Dibenzyl-hydroxymethyl)-pyrrolidins als chirales Hilfsreagenz, so kann der Verlauf des erfindungsgemäßen Verfahrens (Variante b) im Prinzip ebenfalls durch das oben angegebene Reaktionsschema veranschaulicht werden.

Das bei der Durchführung des erfindungsgemäßen Verfahrens als Ausgangsmaterial benötigte E-Isomere des 1-Cyclohexyl-4,4-dimethyl-2-(1,2,4-triazol-1-yl)-pent-1-en-3-ons der Formel (II) ist bereits bekannt (vgl. DE-OS 33 22 181).

Bei der Durchführung des erfindungsgemäßen Verfahrens fungieren entweder Borhydrid (Variante a) oder komplexe Borhydride (Variante b) als Reduktionsmittel. Als komplexes Borhydrid kommt dabei vorzugsweise Natriumboranat in Betracht.

Die bei der Durchführung des erfindungsgemäßen Verfahrens (Variante a) als chirale Hilfsreagenzien benötigten optisch aktiven Prolin-Derivate (= substituierte Hydroxymethyl-pyrrolidine) sind durch die Formel (III) allgemein definiert. In dieser Formel steht $R^1$ vorzugsweise für Alkyl mit 1 bis 6 Kohlenstoffatomen, Phenyl oder Benzyl.

Als Säureadditions-Salze von optisch aktiven Prolin-Derivaten kommen bei der Durchführung der Variante (b) des erfindungsgemäßen Verfahrens vorzugsweise diejenigen Verbindungen in Frage, die durch Anlagerung von Halogenwasserstoffsäuren, wie zum Beispiel Chlorwasserstoff- oder Bromwasserstoffsäure, ferner von Schwefelsäure, Salpetersäure,

Phosphorsäure oder Naphthalin-1,5-disulfonsäure an optisch aktive Verbindungen der Formel (III) gebildet werden.

In den Prolin-Derivaten der Formel (III) beziehungsweise deren Säureadditions-Salzen, die bei der Durchführung des erfindungsgemäßen Verfahrens als chirale Hilfsreagenzien eingesetzt werden, weist das asymmetrisch substituierte Kohlenstoffatom die S-Konfiguration auf.

Die optisch aktiven Prolin-Derivate der Formel (III) sowie deren Säureadditions-Salze sind bisher noch nicht bekannt. Sie lassen sich herstellen, indem man entweder

c) S-Prolinester der Formel

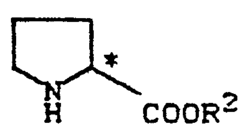

(IV)

in welcher

$R^2$ für Alkyl mit 1 bis 4 Kohlenstoffatomen steht,

mit Grignard-Verbindungen der Formel

$$R^1\text{-Mg X} \qquad (V)$$

in welcher

$R^1$ die oben angegebene Bedeutung hat und

X für Chlor, Brom oder Iod steht,

in Gegenwart eines Verdünnungsmittels umsetzt,

oder

d) S-Prolinester der Formel

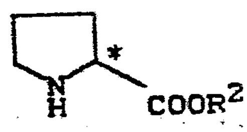

(IV)

in welcher

$R^2$ die oben angegebene Bedeutung hat,

in einer ersten Stufe mit Chlorameisensäure-benzylester der Formel

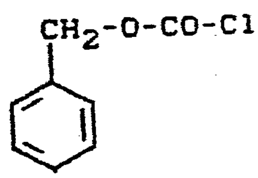

(VI)

in Gegenwart eines Säurebindemittels und in Gegenwart eines Verdünnungsmittels umsetzt,

dann in einer zweiten Stufe die dabei entstehenden, am asymmetrisch substituierten Kohlenstoffatom S-konfigurierten Ester der Formel

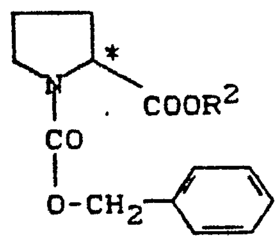

(VII)

in welcher

$R^2$ die oben angegebene Bedeutung hat,

mit Grignard-Verbindungen der Formel

$R^1$-Mg X (V)

in welcher

$R^1$ und X die oben angegebene Bedeutung haben,

in Gegenwart eines Verdünnungsmittels umsetzt und schließlich in einer dritten Stufe die dabei erhaltenen S-Prolin-Derivate der Formel

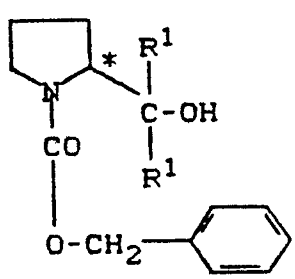

(VIII)

in welcher

$R^1$ die oben angegebene Bedeutung hat,

mit Wasserstoff in Gegenwart eines Katalysators sowie in Gegenwart eines Verdünnungsmittels umsetzt,

und gegebenenfalls noch eine Säure addiert.

Die bei der Herstellung der optisch aktiven Prolin-Derivate der Formel (III) als Ausgangsstoffe benötigten S-Prolinester sind durch die Formel (IV) definiert. In dieser Formel steht $R^2$ vorzugsweise für Methyl oder Ethyl. Es gelangen jeweils diejenigen Verbindungen zum Einsatz, die am asymmetrisch substituierten Kohlenstoffatomen die S-Konfiguration aufweisen.

Die S-Prolinester der Formel (IV) sind bekannt oder lassen sich nach im Prinzip bekannten Verfahren in einfacher Weise herstellen.

Die bei den Verfahren (c) und (d) als Reaktionskomponenten benötigten Grignard-Verbindungen sind durch die Formel (V) allgemein definiert. In dieser Formel hat $R^1$ vorzugsweise diejenigen Bedeutungen, die bereits im Zusammenhang mit der Beschreibung der Verbindungen der Formel (III) vorzugsweise für diesen Rest genannt wurden. X steht für Chlor, Brom oder Iod.

Die Grignard-Verbindungen der Formel (V) sind allgemein bekannte Verbindungen der organischen Chemie. Chlorameisensäurebenzylester der Formel (VI) und dessen Verwendung als Schutzgruppe sind ebenfalls bekannt.

Als Verdünnungsmittel kommen bei der Durchführung des Verfahrens (c) alle üblichen inerten organischen Solventien in Frage. Vorzugsweise verwendbar sind Ether, wie Diethylether, Tetrahydrofuran und Dioxan.

Die Reaktionstemperaturen können bei der Durchführung des Verfahrens (c) in einem bestimmten Bereich variiert werden. Im allgemeinen arbeitet man bei Temperaturen zwischen -10° C und +80° C, vorzugsweise zwischen 0° C und +60° C.

Bei der Durchführung des Verfahrens (c) setzt man auf 1 Mol an S-Prolinester der Formel (IV) 5 bis 10 Mol an Grignard-Verbindung der Formel (V) ein. Die Aufarbeitung erfolgt nach üblichen Methoden. Im allgemeinen geht man so vor, daß man das Reaktionsgemisch mit Wasser hydrolysiert, dann mit wäßriger Ammoniumchlorid-Lösung versetzt, die organische Phase abtrennt, wäscht, trocknet und nach dem Einengen destilliert.

Bei der Durchführung der ersten Stufe des Verfahrens (d) kommen als Säurebindemittel alle üblichen Säureakzeptoren in Betracht. Vorzugsweise verwendbar sind tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der ersten Stufe des Verfahrens (d) alle üblichen inerten organischen Solventien in Frage. Vorzugsweise verwendbar sind Ether, wie Diethylether, Tetrahydrofuran oder Dioxan.

Die Reaktionstemperaturen können bei der Durchführung der ersten Stufe des Verfahrens (d) innerhalb eines bestimmten Bereiches variiert werden. Im allgemeinen arbeitet man bei Temperaturen zwischen -10° C und +60° C, vorzugsweise zwischen 0° C und +50° C.

Bei der Durchführung der ersten Stufe des Verfahrens (d) setzt man auf 1 Mol S-Prolinester der Formel (IV) im allgemeinen 1 bis 1,5 Mol an Chlorameisensäure-benzylester der Formel (VI) sowie 1 bis 1,5 Mol an Säurebindemittel ein. Die Aufarbeitung erfolgt nach üblichen Methoden. Im allgemeinen geht man so vor, daß man das Reaktionsgemisch mit Wasser versetzt, dann mit einem in Wasser wenig löslichen organischen Solvens extrahiert, die vereinigten organischen Phasen wäscht und nach dem Einengen destilliert.

Bei der Durchführung der zweiten Stufe des Verfahrens (d) kommen als Verdünnungsmittel alle üblichen inerten organischen Solventien in Betracht. Vorzugsweise verwendbar sind Ether, wie Diethylether, Tetrahydrofuran und Dioxan.

Die Reaktionstemperaturen können bei der Durchführung der zweiten Stufe des Verfahrens (d) ebenfalls innerhalb eines bestimmten Bereiches variiert werden. Im allgemeinen arbeitet man bei Temperaturen zwischen -10° C und +80° C, vorzugsweise zwischen 0° C und +60° C.

Bei der Durchführung der zweiten Stufe des Verfahrens (d) setzt man auf 1 Mol einer Verbindung der Formel (VII) im

allgemeinen 2 bis 5 Mol an Grignard-Verbindung der Formel (V) ein. Die Aufarbeitung erfolgt nach üblichen Methoden. Im allgemeinen geht man so vor, daß man das Reaktionsgemisch mit Wasser hydrolysiert, dann ansäuert, mit einem mit Wasser wenig mischbaren organischen Solvens extrahiert, die vereinigten organischen Phasen wäscht und nach dem Trocknen einengt.

Bei der Durchführung der dritten Stufe des Verfahrens (d) kommen als Katalysatoren alle für derartige Umsetzungen üblichen Reaktionsbeschleuniger in Frage. Vorzugsweise verwendbar sind Palladium oder Platin auf Kohle.

Als Verdünnungsmittel kommen bei der Durchführung der dritten Stufe des Verfahrens (d) alle üblichen inerten organischen Solventien in Betracht. Vorzugsweise verwendbar sind Alkohole, wie Methanol und Ethanol, ferner Ether, wie Diethylether, Tetrahydrofuran und Dioxan.

Die Reaktionstemperaturen können auch bei der Durchführung der dritten Stufe des Verfahrens (d) innerhalb eines bestimmten Bereiches variiert werden. Im allgemeinen arbeitet man bei Temperaturen zwischen 0 und 40°C, vorzugsweise zwischen 10 und 30°C.

Bei der Durchführung der dritten Stufe des Verfahrens (d) hydriert man mit einem Überschuß an Wasserstoff unter einem Druck von 1 bis 10 bar. Die Aufarbeitung erfolgt nach üblichen Methoden. Im allgemeinen geht man so vor,

daß man den Katalysator abfiltriert und das verbleibende Reaktionsgemisch gegebenenfalls nach vorherigem Ansäuern mit einem in Wasser wenig löslichen organischen Solvens versetzt und das gewünschte Produkt dann durch Abfiltrieren oder Einengen isoliert.

Zur Herstellung von Säureadditions-Salzen der Verbindungen der Formel (III) kommen vorzugsweise diejenigen Säuren in Betracht, die bereits im Zusammenhang mit der Beschreibung diese Salze als vorzugsweise zu addierende Säuren genannt wurden.

Die Säureadditions-Salze der Verbindungen der Formel (III) können in einfacher Weise nach üblichen Salzbildungsmethoden, wie zum Beispiel durch Lösen einer Verbindung der Formel (III) in einem geeigneten inerten Lösungsmittel und Hinzufügen der Säure, wie z.B. Beispiel Chlorwasserstoffsäure, erhalten werden und in bekannter Weise, zum Beispiel durch Abfiltrieren isoliert und gegebenenfalls durch Waschen mit einem inerten organischen Lösungsmittel gereinigt werden. Bei dem oben beschriebenen Verfahren (d) werden Säureadditions-Salze von Verbindungen der Formel (III) vorzugsweise dadurch hergestellt, daß man das nach der Hydrierung anfallende Gemisch nach Entfernung des Katalysators mit der betreffenden Säure und einem inerten organischen Lösungsmittel versetzt und das kristallin ausgeschiedene Salz abfiltriert.

Als Verdünnungsmittel können bei dem erfindungsgemäßen Verfahren zur Herstellung des (-)-Antipoden des (E)-1-

Cyclohexyl-4,4-dimethyl-3-hydroxy-2-(1,2,4-triazol-1-yl)-pent-1-ens der Formel (I) alle für derartige Reaktionen üblichen organischen Solventien eingesetzt werden. Vorzugsweise in Betracht kommen halogenierte Kohlenwasserstoffe, wie Methylenchlorid, ferner Ether, wie Diethylether, tert.-Butylmethylether, Tetrahydrofuran, Dioxan und Anisol.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen -70° C und +60° C, vorzugsweise zwischen -30° C und +50° C.

Das erfindungsgemäße Verfahren wird im allgemeinen unter Normaldruck durchgeführt.

Bei der Durchführung des erfindungsgemäßen Verfahrens setzt man auf 1 Mol an (E)-Isomerem des 1-Cyclohexyl-4,4-dimethyl-2-(1,2,4-triazol-1-yl)-pent-1-en-3-ons der Formel (II) im allgemeinen 1 bis 4 Mol, vorzugsweise 1,3 bis 3 Mol an Borhydrid beziehungsweise an komplexen Borhydrid sowie 1 bis 3 Mol, vorzugsweise 1,2 bis 2 Mol an optisch aktivem Prolin-Derivat der Formel (III) beziehungsweise an einem Säureadditions-Salz eines optisch aktiven Prolin-Derivates der Formel (III) ein.

Im allgemeinen geht man bei der Durchführung des erfindungsgemäßen Verfahrens so vor, daß man das Reduktionsmittel bei Temperaturen zwischen -30° C und +30° C im eine Lösung von optisch aktivem Prolin-Derivat bzw. Säureadditions-Salz von optisch aktivem Prolin-Derivat in einem

organischen Solvens gibt, danach bei Temperaturen zwischen 20 und 50°C das (E)-Isomere des 1-Cyclohexyl-4,4-dimethyl-2-(1,2,4-triazol-1-yl)-pent-1-en-3-ons der Formel (II) hinzufügt und mehrere Stunden bei Temperaturen zwischen 20 und 50°C rührt. Die Aufarbeitung erfolgt nach üblichen Methoden. Im allgemeinen verfährt man in der Weise, daß man das Reaktionsgemisch mit Wasser beziehungsweise Säure und Wasser versetzt, dann mit einem in Wasser wenig löslichen organischen Solvens extrahiert und die vereinigten organischen Phasen gegebenenfalls nach vorherigem Waschen einengt. Der verbleibende Rückstand kann durch Digerieren mit geeigneten organischen Solventien oder durch Umkristallisation oder auch auf chromatographischem Wege weiter gereinigt werden.

Der nach dem erfindungsgemäßen Verfahren herstellbare (-)-Antipode des (E)-1-Cyclohexyl-4,4-dimethyl-3-hydroxy-2-(1,2,4-triazol-1-yl)-pent-1-ens der Formel (I) und dessen Verwendung zur Regulierung des Pflanzenwachstums sind bekannt (vgl. DE-OS 33 02 122).

Das erfindungsgemäße Verfahren wird durch die folgenden Beispiele veranschaulicht.

Herstellungsbeispiele

Beispiel 1

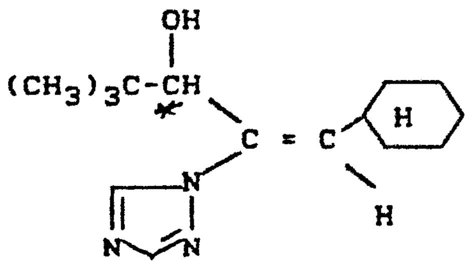

(I)

Eine Lösung von 4,1 g (0,0145 Mol) S-2-(Dibenzyl-hydroxymethyl)-pyrrolidin in 30 ml absolutem Tetrahydrofuran wird bei 20°C unter Rühren mit 29 ml (0,029 Mol) Borwasserstoff-Tetrahydrofuran-Komplex versetzt. Nach 30-minütigem Rühren bei 20°C fügt man 2,61 g (0,01 Mol) an (E)-Isomeren des 1-Cyclohexyl-4,4-dimethyl-2-(1,2,4-triazol-1-yl)-pent-1-en-3-ons hinzu und rührt weitere 16 Stunden bei 40°C. Danach wird das Reaktionsgemisch in 100 ml 2n wäßrige Salzsäure gegossen. Das entstehende Gemisch wird zweimal mit Methylenchlorid extrahiert, und die vereinigten organischen Phasen werden unter vermindertem Druck eingeengt. Der verbleibende Rückstand wird mit einem Gemisch von Chloroform/Essigester = 4:1 an Kieselgel chromatographiert. Man erhält auf diese Weise 2,3 g (87 % der Theorie) an dem (-)-Antipoden des (E)-1-Cyclohexyl-4,4-dimethyl-3-hydroxy-2-(1,2,4-triazol-1-yl)-pent-1-ens in Form eines kristallinen Produktes.

Schmelzpunkt: 150°C

$[\alpha]_D^{20} = -61,4^0$ (c = 0,520 / $CHCl_3$)

Das Produkt besitzt eine optische Reinheit von 83 % e.e.

Beispiel 2

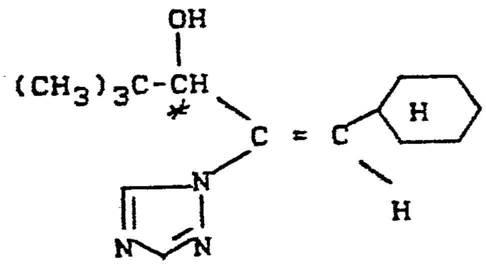

(I)

Eine Suspension von 5,2 g (0,018 Mol) S-2-(Dibenzyl-hydroxymethyl)-pyrrolidin-hydrochlorid in 75 ml absolutem Tetrahydrofuran wird bei -30° C unter Rühren mit 0,68 g (0,018 Mol) Natriumboranat versetzt. Man läßt die Temperatur allmählich auf 20° C ansteigen und rührt noch weitere 2 Stunden bei 20° C. Danach tropft man 3,15 g (0,012 Mol) an (E)-Isomerem des 1-Cyclohexyl-4,4-dimethyl-2-(1,2,4-triazol-1-yl)-pent-1-en-3-ons hinzu und rührt 20 Stunden bei 40° C. Anschließend wird das Reaktionsgemisch in Wasser gegossen. Das entstehende Gemisch wird mit Essigester extrahiert, und die vereinigten organischen Phasen werden unter vermindertem Druck eingeengt. Der verbleibende Rückstand wird mit einem Gemisch von Chloroform/Essigester = 4:1 an Kieselgel chromatographiert. Man erhält auf diese Weise 2,1 g (67 % der Theorie) an dem (-)-Antipoden des (E)-1-Cyclohexyl-4,4-dimethyl-3-hydroxy-2-(1,2,4-triazol-1-yl)-pent-1-ens in Form eines kristallinen Produktes.

Schmelzpunkt: 148-150° C.

$[\alpha]_D^{20}$ : $-62,3^0$ ($c=0,464/CHCl_3$)

Das Produkt besitzt eine optische Reinheit von 79,3 % e.e. (nach chromatographischer Bestimmung).

Beispiel 3

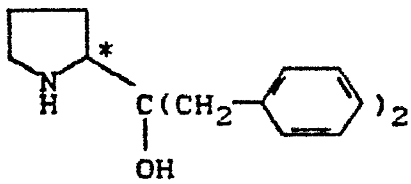

(III-1)

Zu einer aus 25 g (1 Mol) Magnesium und 127 g (1 Mol) Benzylchlorid in 350 ml Ether hergestellten Grignard-Verbindung werden unter Rühren und Eiskühlung 13 g (0,1 Mol) S-Prolinmethylester hinzugetropft. Das Reaktionsgemisch wird erwärmt und 2 Stunden unter Rückfluß gekocht. Danach wird durch langsame Zugabe von Wasser hydrolysiert. Das so entstehende Reaktionsgemisch wird in 1,5 Liter gesättigte wäßrige Ammoniumchlorid-Lösung gegossen. Die organische Phase wird abgetrennt und die wäßrige Phase wird noch einmal mit Ether extrahiert. Die vereinigten organischen Phasen werden mit verdünnter, wäßriger Natronlauge gewaschen, dann getrocknet und unter vermindertem Druck eingeengt. Der verbleibende Rückstand wird im Hochvakuum destilliert. Man erhält auf diese Weise 19,7 g (70 % der Theorie) an S-2-(Dibenzyl-hydroxymethyl)-pyrrolidin in Form einer Flüssigkeit.

Siedepunkt: 165-170° C / 0,03 mbar

$[\alpha]_D^{20}$ = +9,6° (c = 0,581 / $CH_3OH$)

Beispiel 4

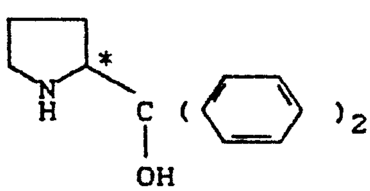

(III-2)

Zu einer aus 25 g (1 Mol) Magnesium und 157 g (1 Mol) Brombenzol in 350 ml Ether hergestellten Grignard-Verbindung werden unter Rühren und Eiskühlung 13 g (0,1 Mol) S-Prolinmethylester hinzugetropft. Das Reaktionsgemisch wird erwärmt und 2 Stunden unter Rückfluß gekocht. Danach wird durch langsame Zugabe von Wasser hydrolysiert. Das so entstehende Reaktionsgemisch wird in 1,5 Liter gesättigte, wäßrige Ammoniumchlorid-Lösung gegossen. Die vereinigten organischen Phasen werden mit verdünnter, wäßriger Natronlauge gewaschen, dann getrocknet und unter vermindertem Druck eingeengt. Der verbleibende Rückstand wird mit Essigester an Kieselgel chromatographiert. Man erhält auf diese Weise 10,8 g (22 % der Theorie) an S-2-(Diphenyl-hydroxymethyl)-pyrrolidin in Form eines viskosen Produktes.

Beispiel 5

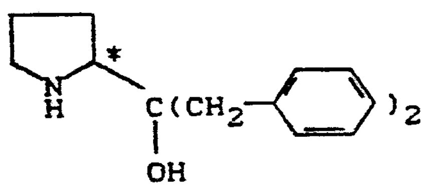

x HCl (III-3)

1. Stufe:

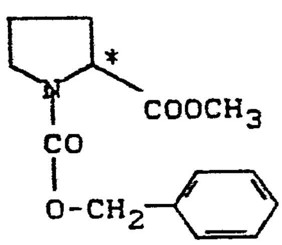

(VII-1)

In eine Lösung von 130 g (1 Mol) S-Prolinmethylester und

155 ml (1,1 Mol) Triethylamin in 600 ml absolutem Tetrahydrofuran werden bei einer Temperatur zwischen 0° C und 10° C unter Rühren 180 g (1,01 Mol ) Chlorameisensäurebenzylester getropft, wobei sich ein farbloser Niederschlag abscheidet. Man läßt das Reaktionsgemisch 16 Stunden bei Raumtemperatur stehen und gießt es dann in 1,5 Liter Wasser. Das entstehende Gemisch wird mehrfach mit Essigester extrahiert. Die vereinigten organischen Phasen werden zunächst mit verdünner, wäßriger Salzsäure, dann mit Wasser gewaschen und unter vermindertem Druck eingeengt. Der verbleibende Rückstand wird im Hochvakuum destilliert. Man erhält auf diese Weise 188 g (72 % der Theorie) an N-Benzyloxycarbonyl-S-prolinmethylester vom Siedepunkt 160° C / 0,1 mbar.

2. Stufe:

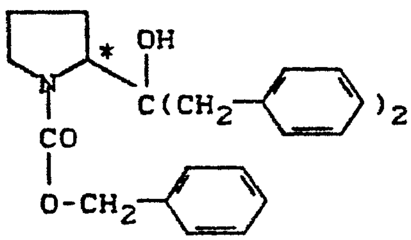

(VIII-1)

Zu einer aus 28 g (1 Mol) Magnesium und 171 g (1 Mol) Benzylbromid in 400 ml absolutem Ether hergestellten Grignard-Verbindung wird unter Rühren bei 0° C eine Lösung von 87 g (0,33 mol) N-Benzyloxycarbonyl-S-prolinmethylester in 200 ml absolutem Ether hinzugetropft. Man läßt das Reaktionsgemisch 16 Stunden bei Raumtemperatur stehen und hydrolysiert dann durch Zugabe von Wasser. Anschließend wird mit konzentrierter Salzsäure angesäuert und mit

Essigester extrahiert. Die vereinigten organischen Phasen werden mit Wasser gewaschen, getrocknet und unter vermindertem Druck eingeengt. Man erhält auf diese Weise 163 g eines gelben Öles, das gemäß Gaschromatogramm zu 50 % aus N-Benzyloxycarbonyl-S-2-(dibenzyl-hydroxymethyl)-pyrrolidin besteht. Die Ausbeute errechnet sich danach zu 59,9 % der Theorie.

3. Stufe:

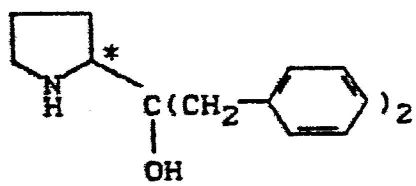 x HCl (III-3)

Eine Lösung von 75 g des Rohproduktes mit einem Gehalt von 50 % an N-Benzyloxycarbonyl-S-2-(dibenzyl-hydroxymethyl)-pyrrolidin in 300 ml Ethanol wird mit 10 g Palladium auf Kohle (5 %ig) 5 Stunden bei 20°C mit Wasserstoff unter einem Druck von 5 bar hydriert. Nach dem Absaugen des Katalysators wird unter Kühlung ein starker Chlorwasserstoff-Strom in die Lösung eingeleitet. Anschließend wird das Reaktionsgemisch durch Abziehen des Lösungsmittels unter vermindertem Druck eingeengt, und das verbleibende ölige Produkt wird mit 300 ml Essigester versetzt. Der dabei auskristallisierende Niederschlag wird abgesaugt. Man erhält auf diese Weise 25 g (87,5 % der Theorie, bezogen auf 50 %iges Ausgangsprodukt) an S-2-(Dibenzyl-hydroxymethyl)-pyrrolidin-hydrochlorid.
Schmelzpunkt: 130°C.

Durch Behandlung des so hergestellten Salzes mit verdünnter wäßriger Natronlauge erhält man das freie S-2-(Dibenzyl-hydroxymethyl)-pyrrolidin, das mit dem im Beispiel 3 beschriebenen Produkt identisch ist.

Patentansprüche

1. Verfahren zur Herstellung des (-)-Antipoden des (E)-1-Cyclohexyl-4,4-dimethyl-3-hydroxy-2-(1,2,4-triazol-1-yl)-pent-1-ens der Formel

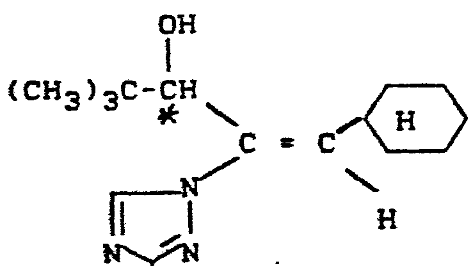

(I)

dadurch gekennzeichnet, daß man (E)-Isomeres des 1-Cyclohexyl-4,4-dimethyl-2-(1,2,4-triazol-1-yl)-pent-1-en-3-ons der Formel

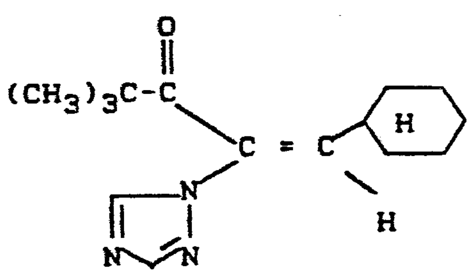

(II)

a) mit Borhydrid in Gegenwart eines optisch aktiven Prolin-Derivates der Formel

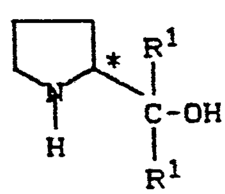

(III)

in welcher

$R^1$ für Alkyl, Phenyl oder Benzyl steht,

oder

b) mit komplexen Borhydriden in Gegenwart eines Säureadditions-Salzes eines optisch aktiven Prolin-Derivates der Formel (III)

in Gegenwart eines Verdünnungsmittels bei Temperaturen zwischen -70°C und +60°C umsetzt.

2. Verfahren gemäß Anspruch 1, dadurch gekennzeichnet, daß man als optisch aktives Prolin-Derivat der Formel (III) S-2-(Dibenzyl-hydroxymethyl)-pyrrolidin einsetzt.

3. Verfahren gemäß Anspruch 1, dadurch gekennzeichnet, daß man als optisch aktives Prolin-Derivat der Formel (III) S-2-(Dibenzyl-hydroxymethyl)-pyrrolidin-hydrochlorid einsetzt.

4. Verfahren gemäß Anspruch 1, dadurch gekennzeichnet, daß man als komplexes Borhydrid Natriumboranat einsetzt.

5. Verfahren gemäß Anspruch 1, dadurch gekennzeichnet, daß man die Umsetzung bei Temperaturen zwischen -30°C und +50°C durchführt.

6. Optisch aktive Prolin-Derivate der Formel

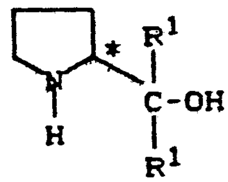

(III)

in welcher
$R^1$ für Alkyl, Phenyl oder Benzyl steht,

sowie deren Säureadditions-Salze.

7. Verfahren zur Herstellung von optisch aktiven Prolin-Derivaten der Formel

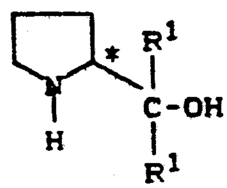

(III)

in welcher
$R^1$ für Alkyl, Phenyl oder Benzyl steht,

sowie von deren Säureadditions-Salzen, dadurch gekennzeichnet, daß man entweder

c) S-Prolinester der Formel

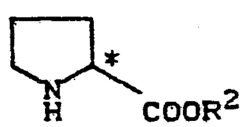

(IV)

0237902

in welcher

$R^2$ für Alkyl mit 1 bis 4 Kohlenstoffatomen steht,

mit Grignard-Verbindungen der Formel

$$R^1\text{-Mg X} \qquad (V)$$

in welcher

$R^1$ die oben angegebene Bedeutung hat und

X für Chlor, Brom oder Iod steht,

in Gegenwart eines Verdünnungsmittels umsetzt,

oder

d) S-Prolinester der Formel

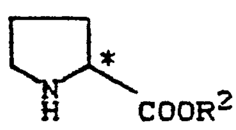

(IV)

in welcher

$R^2$ die oben angegebene Bedeutung hat,

in einer ersten Stufe mit Chlorameisensäure-benzylester der Formel

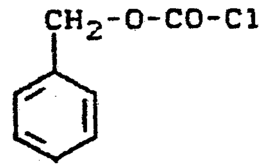

(VI)

in Gegenwart eines Säurebindemittels und in Gegenwart eines Verdünnungsmittels umsetzt,

dann in einer zweiten Stufe die dabei entstehenden, am asymmetrisch substituierten Kohlenstoffatom S-konfigurierten Ester der Formel

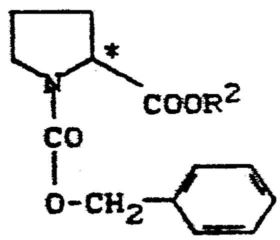

(VII)

in welcher

$R^2$ die oben angegebene Bedeutung hat,

mit Grignard-Verbindungen der Formel

$R^1$-Mg X        (V)

in welcher

$R^1$ und X die oben angegebene Bedeutung haben,

in Gegenwart eines Verdünnungsmittels umsetzt und schließlich in einer dritten Stufe die dabei erhaltenen S-Prolin-Derivate der Formel

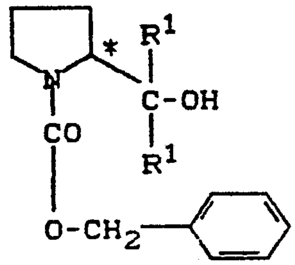

(VIII)

in welcher

$R^1$ die oben angegebene Bedeutung hat,

mit Wasserstoff in Gegenwart eines Katalysators sowie in Gegenwart eines Verdünnungsmittels umsetzt,

und gegebenenfalls noch eine Säure addiert.